(19) Europäisches Patentamt
European Patent Office
Office européen des brevets

(11) Numéro de publication : **0 136 230**
**B1**

(12) **FASCICULE DE BREVET EUROPÉEN**

(45) Date de publication du fascicule du brevet : **29.07.87**

(51) Int. Cl.⁴ : **C 07 C 15/16**, C 07 C 2/86

(21) Numéro de dépôt : **84401832.5**

(22) Date de dépôt : **14.09.84**

(54) **Compositions d'oligomères de polyarylalcanes et leur procédé de fabrication.**

(30) Priorité : **23.09.83 FR 8315127**

(43) Date de publication de la demande : **03.04.85 Bulletin 85/14**

(45) Mention de la délivrance du brevet : **29.07.87 Bulletin 87/31**

(84) Etats contractants désignés : **AT BE CH DE FR GB IT LI LU NL SE**

(56) Documents cités : **DE-A- 3 127 905**

(73) Titulaire : **ATOCHEM**
**4 & 8, Cours Michelet La Défense 10**
**F-92800 Puteaux (FR)**

(72) Inventeur : **Commandeur, Raymond**
**Le Rocher Avenue de Venaria**
**F-38220 Vizille (FR)**
Inventeur : **Gurtner, Bernard**
**5 Boulevard Agutte Sembat**
**F-38000 Grenoble (FR)**
Inventeur : **Gervason, Pierre**
**5, Rue de Basly**
**F-92230 Genevilliers (FR)**

(74) Mandataire : **Foiret, Claude et al**
**ATOCHEM Département Propriété Industrielle**
**F-92091 Paris la Défense 10 Cédex 42 (FR)**

**EP 0 136 230 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouvelles compositions d'oligomères de polyarylalcanes et leur procédé de fabrication.

Ces compositions d'oligomères de polyarylalcanes sont constituées du mélange de deux oligomères A et B. Elles sont caractérisées en ce que :

L'oligomère A est un mélange d'isomères de formule

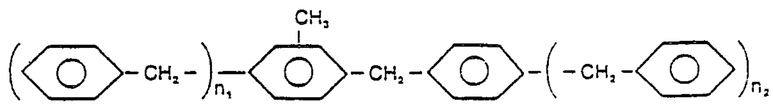

avec $n_1$ et $n_2 = 0,1$, et 2, sachant que $n_1 + n_2 \leqslant 3$ et l'oligomère B est un mélange d'isomères de formule

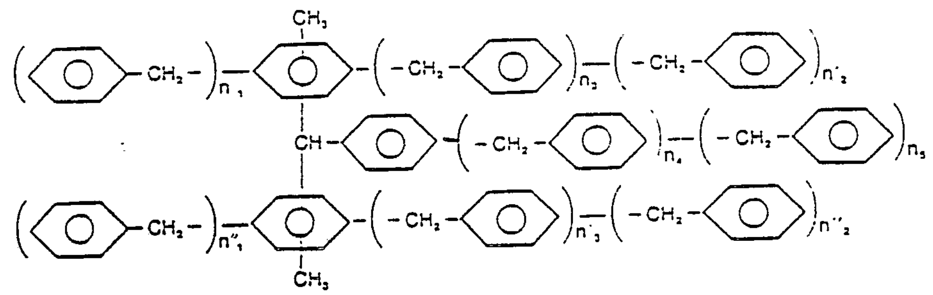

avec

$n'_1$, $n''_1$ et $n_4 = 0,1$ et 2

$n'_2$, $n''_2$, $n_3$, $n'_3$ et $n_5 = 0$ et 1

sachant que $n'_1 + n''_1 + n'_2 + n''_2 + n_3 + n'_3 + n_4 + n_5 \leqslant 2$.

Ce mélange d'oligomères de polyarylalcanes composé d'une part de benzyltoluène, de dibenzyltoluène ainsi que d'homologues supérieurs et d'autre part de ditolylphénylméthane et d'homologues supérieurs peut avoir les mêmes applications que le monobenzyltoluène avec les avantages de ces produits sans en posséder les inconvénients. Par exemple, en tant que diélectrique pour condensateur le monobenzyltoluène pur est intéressant, mais il a l'inconvénient de cristalliser à —20 °C, après une surfusion pouvant être longue, ce qui le rend inutilisable dans les pays froids. Dans la même application, le dibenzyltoluène ne présente pas cet inconvénient mais sa viscosité aux basses températures reste trop importante pour le rendre utilisable. Les compositions selon l'invention présentent non seulement une viscosité toujours compatible avec l'application visée mais encore sont obtenues sans faire appel à des procédés de fabrication sophistiqués ou de séparation suivie d'une recombinaison.

Les inconvénients du mono et dibenzyltoluène étant connus de longue date, de nombreux mélanges de remplacement ont été proposés à partir d'oligomères de polyarylalcanes. Il est cependant toujours difficile de concilier la qualité des propriétés des produits obtenus avec l'aspect économique des moyens d'obtention de ces produits de remplacement.

C'est ainsi que dans la DOS 3 127 905 est décrit un procédé de synthèse faisant appel, de préférence, à la condensation du chlorure de benzyle et du toluène. Cette réaction de condensation est peu sélective en monobenzyltoluène ou en mélange benzyltoluène et diphénylméthane, les rendements pondéraux étant généralement inférieurs à 70 %. Dans ce même document est également décrit un procédé de synthèse faisant appel à la condensation entre le formaldéhyde et les composés aromatiques, cette réaction a un rendement pondéral faible et pour les oligomères conduit à des réactions secondaires. En effet, on utilise quatre matières premières, à savoir le benzène, le toluène, le formol et l'acide sulfurique : à côté d'oligomères du type A, réaction du benzène, du toluène et du formol, on forme des oligomères du type diphénylméthane, réaction du benzène et du formol et du type ditolylméthane, réaction du toluène et du formol.

Enfin, dans le brevet japonais 55-5689 est cité l'emploi, en tant que fluide diélectrique, des isomères suivants :

2,4-dibenzyltoluène
2,6-dibenzyltoluène
O-benzyltoluène
p-benzyltoluène

pris isolément ou en mélange. Outre le fait que la synthèse de ces isomères nécessite des réactions spécifiques, p-benzyltoluène par exemple à partir de chlorure de p-méthylbenzyle et de benzène, ou des séparations coûteuses, il faut encore par la suite procéder à un mélange de ces isomères, ce qui n'est guère intéressant sur le plan économique.

Il faut également rappeler que l'emploi du chlorure de benzyle comme matière première de condensation et son stockage ne sont pas sans présenter quelques inconvénients dus à la grande réactivité du chlore benzylique, ce qui explique aussi son prix élevé par rapport à d'autres dérivés aromatiques chlorés.

De façon générale, on n'a réussi à mettre au point des produits particulièrement recherchés pour l'application diélectrique, comme le monobenzyltoluène ou encore le dibenzyltoluène, qu'en utilisant des synthèses peu sélectives pour l'obtention de ces produits.

Les compositions d'oligomères de polyarylalcanes selon l'invention sont obtenues en mettant en œuvre un procédé de synthèse faisant appel, non pas à la réaction du chlorure de benzyle sur un composé aromatique mais simplement à la réaction du chlore sur le toluène.

Le procédé d'obtention des compositions d'oligomères de polyarylalcanes est caractérisé en ce que, dans une première étape, on fait réagir le chlore sur le toluène par réaction radicalaire en présence de générateur de radicaux libres et dans une seconde étape on soumet le produit de réaction de cette première étape à l'action d'un halogénure minéral ou d'un acide minéral.

La chloration radicalaire du toluène est habituellement réalisée à une température comprise entre 50 et 110 °C et mieux entre 70 et 100 °C. Elle est de préférence menée de telle sorte qu'on ne transforme que 10 à 30 %, exprimé en pourcentage molaire, du toluène engagé en dérivé chloré correspondant. En tant que générateur de radicaux libres, on peut employer soit une initiation photochimique, soit un initiateur chimique ; parmi les initiateurs chimiques, on peut citer les composé azo comme l'azodiisobutyronitrile ou encore l'azodivaléronitrile, comme par exemple le peroxyde de lauroyle. La quantité d'initiateur chimique mise en œuvre est généralement comprise entre 0,05 et 3 % en poids par rapport au toluène engagé, et de préférence entre 0,1 et 1,5 %.

Le milieu réactionnel obtenu durant l'étape précédente est ensuite soumis à l'action d'un halogénure minéral, ou encore d'un acide minéral. Cette réaction a lieu en pratique à une température comprise entre 30 et 110 °C, et de préférence entre 50 et 100 °C. Parmi les halogénures minéraux, on peut utiliser le chlorure ferrique, le trichlorure d'antimoine, le tétrachlorure de titane ou encore le chlorure d'aluminium à des teneurs pondérales par rapport au milieu réactionnel compris habituellement entre 50 ppm et 1 % et de préférence entre 100 ppm et 0,5 %. Les acides minéraux peuvent étalement être utilisés : l'acide sulfurique par exemple à une concentration pondérale comprise entre 70 et 95 %. Il est aussi possible d'employer les zéolithes ou encore certains oxydes minéraux. Une variante du procédé au niveau de cette deuxième étape consiste à couler le mélange réactionnel de la première phase dans un pied de toluène, ou de toluène et du mélange d'oligomères selon l'invention, contenant l'halogénure ou l'acide minéral sous forme de solution ou de dispersion ; cette variante est particulièrement intéressante pour la mise en continu d'un tel procédé car il est bien entendu que cette synthèse est réalisable en discontinu et en continu.

Il est recommandé, après distillation du toluène en excès, de procéder à l'élimination de l'halogénure minéral ou de l'acide minéral par toute technique connue telle que : lavage à l'eau, neutralisation, séchage.

Selon le procédé décrit on obtient, de façon courante, directement le mélange d'oligomères de polyarylalcanes dans les proportions pondérales suivantes :

Composé A en mélange d'isomères :

$n_1 + n_2 = 0$ compris entre 56 et 90 %
$n_1 + n_2 = 1$ compris entre 7 et 28 %
$n_1 + n_2 = 2$ compris entre 1,5 et 8 %
$n_1 + n_2 = 3$ compris entre 0,1 et 1 %

Composé B en mélange d'isomères :

$n'_1 + n''_1 + n'_2 + n''_2 + n_3 + n'_3 + n_4 + n_5 = 0$ compris entre 1,1 et 5 %
$n'_1 + n''_1 + n'_2 + n''_2 + n_3 + n'_3 + n_4 + n_5 = 1$ compris entre 0,25 et 1,5 %
$n'_1 + n''_1 + n'_2 + n''_2 + n_3 + n'_3 + n_4 + n_5 = 2$ compris entre 0,05 et 0,5 %.

Suivant l'usage applicatif du mélange d'oligomères de polyarylalcanes selon l'invention, il peut être intéressant de procéder à une évaporation-flash de ce mélange pour éliminer les traces d'impuretés provenant soit des matières premières, soit du procédé, ou ayant une origine accidentelle ; dans tous les cas, leurs teneurs pondérales ne dépassent pas 1 à 2 %. Parmi les appareils utilisables, on préférera un évaporateur à film mince ; il faut signaler cependant qu'au niveau industriel, les possibilités techniques de tels appareils au niveau de la tenue au vide ne permettent pas toujours de récupérer l'intégralité du mélange d'oligomères de polyarylalcanes : ces produits évaporés font néanmoins partie intégrante de l'invention, comme c'est le cas en particulier des isomères du composé A pour $n_1 + n_2 = 3$ et du composé B pour

$$n'_1 + n''_1 + n'_2 + n''_2 + n_3 + n'_3 + n_4 + n_5 = 2.$$

Les mélanges d'oligomères sont obtenus, selon le procédé décrit, avec des rendements pondéraux exprimés par rapport au toluène réagi, pouvant même atteindre 98 % dans les meilleures conditions.

Les exemples suivants illustrent l'invention sans la limiter. La composition des milieux réactionnels est définie : par distillation sous vide de 2 mm de Hg (267 Pa)

| | |
|---|---|
| A : $n_1 + n_2 = 0$ | Ebullition = 90-130 °C |
| A : $n_1 + n_2 = 1$<br>B : $n'_1 + n''_1 + n'_2 + n''_2 + n_3 + n'_3 + n_4 + n_5 = 0$ | Ebullition = 130-200 °C |
| A : $n_1 + n_2 = 2$<br>B : $n'_1 + n''_1 + n'_2 + n''_2 + n_3 + n'_3 + n_4 + n_5 = 1$ | Ebullition = 200-250 °C |
| A : $n_1 + n_2 = 3$<br>B : $n'_1 + n''_1 + n'_2 + n''_2 + n_3 + n'_3 + n_4 + n_5 \leq 2$ | Résidu de distillation |

et par détermination de la teneur en composé B dans chacune des fractions de distillation par RMN du proton en milieu $CDCl_3$ avec le tétramétylsilane comme référence. Le proton du groupement CH correspondant aux dérivés du triphénylméthane a un déplacement chimique à 5,5 ppm.

Exemple 1

Dans un réacteur muni d'une agitation, d'un condenseur, d'un tube d'alimentation de chlore et d'une lampe PHILIPS TLADK de 30 Watts, on place 368 g de toluène (3,8 moles) ; on introduit ensuite 71 g de chlore gazeux (1 mole) en maintenant la température à 80 °C durant une heure. Après arrêt de l'initiation photochimique, le milieu réactionnel est placé dans une ampoule de coulage et il est introduit en une heure dans un réacteur muni d'une agitation, contenant 0,2 mole de toluène et 60 mg de $FeCl_3$, à une température de 100 °C. L'ensemble est maintenu encore 1 heure à 100 °C sous agitation après fin de coulage.

Le milieu réactionnel, après refroidissement, est lavé à l'acide chlorhydrique 10 %, puis à l'eau jusqu'à neutralité, et enfin le toluène en excès éliminé par distillation. Le mélange d'oligomères de polyarylalcanes obtenu a la composition pondérale suivante :

| PRODUIT | $n_1 + n_2$ | | | | $n'_1+n''_1+n'_2+n''_2+n_3+n'_3+n_4+n_5$ | | |
|---|---|---|---|---|---|---|---|
| | 0 | 1 | 2 | 3 | 0 | 1 | 2 |
| A | 64 % | 21,8 % | 7,8 % | 2,8 % | - | - | - |
| B | - | - | - | - | 2,5 % | 0,9 % | 0,3 % |

Pour une viscosité :
à 50 °C = 6,5 centistokes
20 °C = 10,5 centistokes
— 20 °C = 33 centistokes.

Le rendement pondéral, calculé par rapport au toluène réagi, est de 98 %.

Ce produit, soumis à une évaporation-flash à 300 °C sous 2 mm de Hg (267 Pa) donne, avec un rendement de 95 %, un mélange d'oligomères de polyarylalcanes dont la seule différence avec la composition donnée ci-dessus est l'absence des produits correspondant à :

A et $n_1 + n_2 = 3$

B et $n'_1 + n''_1 + n'_2 + n''_2 + n_3 + n'_3 + n_4 + n_5 = 2$.

Exemple 2

En opérant dans les mêmes conditions opératoires qu'à l'exemple 1, mais en engageant 552 g de toluène (5,8 moles) pour 71 g de chlore (1 mole) et ensuite 100 mg de $FeCl_3$ et 0,2 mole de toluène à la réaction de couplage, on obtient un mélange d'oligomères de polyarylalcanes ayant la composition

pondérale suivante :

| PRODUIT | $n_1 + n_2$ | | | | $n'_1+n''_1+n'_2+n''_2+n_3+n'_3+n_4+n_5$ | | |
|---|---|---|---|---|---|---|---|
| | 0 | 1 | 2 | 3 | 0 | 1 | 2 |
| A | 77 % | 15,5 % | 4 % | 0,9 % | - | - | - |
| B | - | - | - | - | 2 % | 0,5 % | 0,1 % |

Pour une viscosité :
à 50 °C = 2,8 centistokes
20 °C = 6,5 centistokes
— 30 °C = 100 centistokes.

**Revendications**

1. Compositions d'oligomères de polyarylalcanes constituées du mélange de deux oligomères A et B caractérisées en ce que : l'oligomère A est un mélange d'isomères de formule

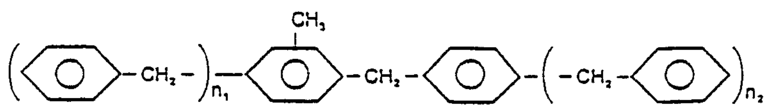

avec $n_1$ et $n_2$ = 0, 1 et 2, sachant que $n_1 + n_2 \leq 3$ et l'oligomère B est un mélange d'isomères de formule

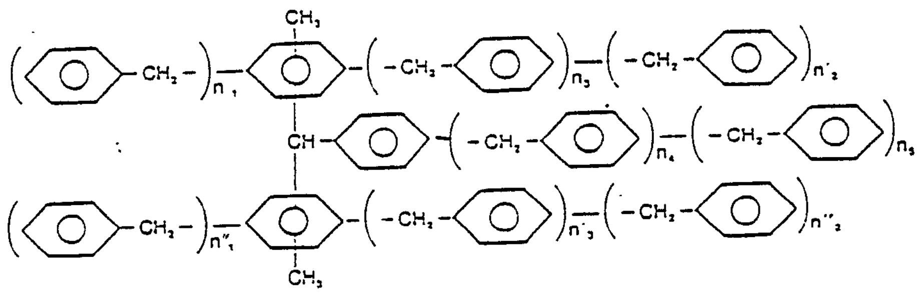

avec
$n'_1$, $n''_1$ et $n_4$ = 0, 1 et 2
$n'_2$, $n''_2$, $n_3$, $n'_3$ et $n_5$ = 0 et 1
sachant que $n'_1 + n''_1 + n'_2 + n''_2 + n_3 + n'_3 + n_4 + n_5 \leq 2$.

2. Compositions selon la revendication 1 caractérisées en ce que le mélange d'oligomères se trouve dans les proportions pondérales de :

Composé A en mélange d'isomères :

$n_1 + n_2 = 0$ compris entre 56 et 90 %
$n_1 + n_2 = 1$ compris entre 7 et 28 %
$n_1 + n_2 = 2$ compris entre 1,5 et 8 %
$n_1 + n_2 = 3$ compris entre 0,1 et 1 %

et composé B en mélange d'isomères :

$n'_1 + n''_1 + n'_2 + n''_2 + n_3 + n'_3 + n_4 + n_5 = 0$ compris entre 1,1 et 5 %

$n'_1 + n''_1 + n'_2 + n''_2 + n_3 + n'_3 + n_4 + n_5 = 1$ compris entre 0,25 et 1,5 %
$n'_1 + n''_1 + n'_2 + n''_2 + n_3 + n'_3 + n_4 + n_5 = 2$ compris entre 0,05 et 0,5 %.

3. Compositions selon la revendication 2 caractérisées en ce que le mélange d'oligomères se trouve dans les proportions pondérales de :

Composé A en mélange d'isomères :

$n_1 + n_2 = 0$ compris entre 56 et 90 %
$n_1 + n_2 = 1$ compris entre 7 et 28 %
$n_1 + n_2 = 2$ compris entre 1,5 et 8 %

et composé B en mélange d'isomères :

$n'_1 + n''_1 + n'_2 + n''_2 + n_3 + n'_3 + n_4 + n_5 = 0$ compris entre 1,1 et 5 %
$n'_1 + n''_1 + n'_2 + n''_2 + n_3 + n'_3 + n_4 + n_5 = 1$ compris entre 0,25 et 1,5 %.

4. Procédé de fabrication des compositions d'oligomères de polyarylalcanes des revendications 1 à 3 caractérisé en ce que, dans une première étape on fait réagir du chlore sur du toluène par réaction radicalaire en présence de générateur de radicaux libres et en ce que dans une seconde étape on soumet le produit de réaction de cette première étape à l'action d'un halogénure minéral ou d'un acide minéral.

5. Procédé selon la revendication 4 caractérisé en ce que dans la première étape on ne transforme que 10 à 30 % molaire du toluène engagé en dérivé chloré.

6. Procédé selon l'une des revendications 4 à 5 caractérisé en ce qu'on procède à une évaporation-flash sur le produit obtenu en seconde étape.

**Claims**

1. Polyarylalkane oligomer compositions consisting of the mixture of two oligomers A and B, characterized in that: the oligomer A is a mixture of isomers of formula

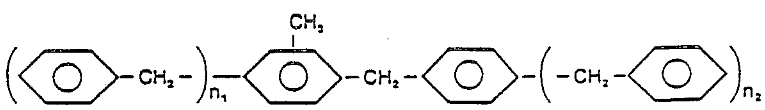

with $n_1$ and $n_2 = 0$, 1 and 2, given that $n_1 + n_2 \leq 3$ and the oligomer B is a mixture of isomers of the formula

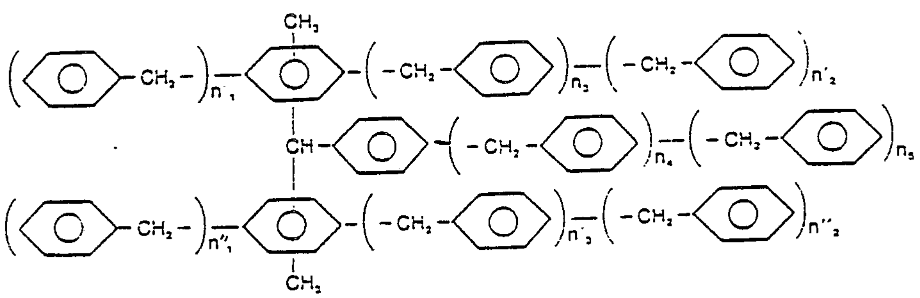

with
$n'_1$, $n''_1$ and $n_4 = 0$, 1 and 2
$n'_2$, $n''_2$, $n_3$, $n'_3$ and $n_5 = 0$ and 1
given that $n'_1 + n''_1 + n'_2 + n''_2 + n_3 + n'_3 + n_4 + n_5 \leq 2$.

2. Compositions according to Claim 1, characterized in that the mixture of oligomers is present in the following proportions by weight :

Compound A as a mixture of isomers :

$n_1 + n_2 = 0$ between 56 and 90 %
$n_1 + n_2 = 1$ between 7 and 28 %
$n_1 + n_2 = 2$ between 1.5 and 8 %
$n_1 + n_2 = 3$ between 0.1 and 1 %

and compound B as a mixture of isomers :

$n'_1 + n''_1 + n'_2 + n''_2 + n_3 + n'_3 + n_4 + n_5 = 0$ between 1.1 and 5 %
$n'_1 + n''_1 + n'_2 + n''_2 + n_3 + n'_3 + n_4 + n_5 = 1$ between 0.25 and 1.5 %
$n'_1 + n''_1 + n'_2 + n''_2 + n_3 + n'_3 + n_4 + n_5 = 2$ between 0.05 and 0,5 %.

3. Compositions according to Claim 2, characterized in that the mixture of oligomers is present in the following proportions by weight :

Compound A as a mixture of isomers :

$n_1 + n_2 = 0$ between 56 and 90 %
$n_1 + n_2 = 1$ between 7 and 28 %
$n_1 + n_2 = 2$ between 1.5 and 8 %

and compound B as a mixture of isomers :

$n'_1 + n''_1 + n'_2 + n''_2 + n_3 + n'_3 + n_4 + n_5 = 0$ between 1.1 and 5 %
$n'_1 + n''1 + n'_2 + n''_2 + n_3 + n'_3 + n_4 + n_5 = 1$ between 0.25 and 1,5 %.

4. Process for manufacturing the polyarylalkane oligomer compositions of Claims 1 to 3, characterized in that, in a first stage, chlorine is reacted with toluene by a free-radical reaction in the presence of a free-radical generator, and in that, in a second stage, the reaction product of this first stage is subjected to the action of an inorganic halide or an inorganic acid.

5. Process according to Claim 4, characterized in that, in the first stage, only 10 to 30 mole % of the toluene introduced is converted to a chlorinated derivative.

6. Process according to one of Claims 4 to 5, characterized in that a flash evaporation is performed on the product obtained in the second stage.

**Patentansprüche**

1. Zusammensetzungen von Oligomeren aus Polyarylalkanen bestehend aus eine einem Gemisch von zwei Oligomeren A und B, dadurch gekennzeichnet, daß das Oligomere A eine Mischung von Isomeren der Formel

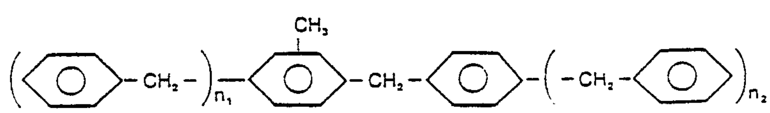

mit $n_1$ und $n_2 = 0$, 1 und 2 ist, wobei $n_1 + n_2 \leq 3$ ist und daß das Oligomere B eine Mischung von Isomeren der Formel

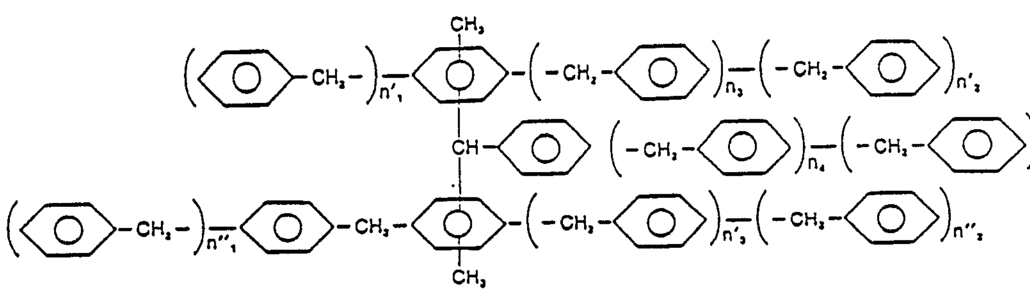

mit

$n'_1$, $n''_1$ und $n_4 = 0$, 1 und 2
$n'_2$, $n''_2$, $n_3$, $n'_3$ und $n_5 = 0$ und 1 ist,
wobei $n'_1 + n''_1 + n'_2 + n''_2 + n_3 + n'_3 + n_4 + n_5 \leq 2$ ist.

2. Zusammensetzungen nach Anspruch 1, dadurch gekennzeichnet, daß das Oligomerengemisch die folgenden Gewichtsverhältnisse aufweist :

Verbindung A als Gemisch der Isomeren :

$n_1 + n_2 = 0$ zwischen 56 und 90 %
$n_1 + n_2 = 1$ zwischen 7 und 28 %

$n_1 + n_2 = 2$ zwischen 1,5 und 8 %
$n_1 + n_2 = 3$ zwischen 0,1 und 1 %

und Verbindung B als Gemisch der Isomeren :

$n'_1 + n''_1 + n'_2 + n''_2 + n_3 + n'_3 + n_4 + n_5 = 0$ zwischen 1,1 und 5 %
$n'_1 + n''_1 + n'_2 + n''_2 + n_3 + n'_3 + n_4 + n_5 = 1$ zwischen 0,25 und 1,5 %
$n'_1 + n''_1 + n'_2 + n''_2 + n_3 + n'_3 + n_4 + n_5 = 2$ zwischen 0,05 und 0,5 %.

3. Zusammensetzungen nach Anspruch 2, dadurch gekennzeichnet, daß das Oligomerengemisch die folgenden Gewichtzverhältnisse aufweist :

Verbindung A als Gemisch der Isomeren :

$n_1 + n_2 = 0$ zwischen 56 und 90 %
$n_1 + n_2 = 1$ zwischen 7 und 28 %
$n_1 + n_2 = 2$ zwischen 1,5 und 8 %

und Verbindung B als Gemisch der Isomeren :

$n'_1 + n''_1 + n'_2 + n''_2 + n_3 + n'_3 + n_4 + n_5 = 0$ zwischen 1,1 und 5 %
$n'_1 + n''_1 + n'_2 + n''_2 + n_3 + n'_3 + n_4 + n_5 = 1$ zwischen 0,25 und 1,5 %.

4. Verfahren zur Herstellung der Zusammensetzungen von Oligomeren aus Polyarylalkanen nach den Ansprüchen 1 bis 3, dadurch gekennzeichnet, daß man in einem ersten Schritt durch radikalische Reaktion Chlor auf Toluol in Gegenwart eines Bildners freier Radikale einwirken läßt und daß man in einem zweiten Schritt das Reaktionsprodukt dieses ersten Schritts der Einwirkung eines anorganischen Halogenids oder einer Mineralsäure aussetzt.

5. Verfahren nach Anspruch 4, dadurch gekennzeichnet, daß man im ersten Schritt nur 10 bis 30 Mol-% des eingesetzten Toluols in das Chlorderivat umwandelt.

6. Verfahren nach einem der Ansprüche 4 bis 5, dadurch gekennzeichnet, daß man das im zweiten Schritt erhaltene Produkt einer Flash-Verdampfung unterwirft.